# EUROPEAN PATENT SPECIFICATION

(11) **EP 1 163 844 B1**
(45) Date of publication and mention of the grant of the patent: **29.08.2007**
(21) Application number: 00905311.7
(22) Date of filing: 24.02.2000
(51) Int. Cl.: A01K 67/033, A01K 61/00, C12N 15/11

(54) **TRANSGENIC MOLLUSC AND METHOD FOR CONSTRUCTING THE SAME**
TRANSGENER MOLLUSK UND VERFAHREN ZU SEINER HERSTELLUNG
MOLLUSQUE TRANSGENIQUE ET SON PROCEDE DE CREATION

(30) Priority: 25.02.1999 JP 4844499
(43) Date of publication of application: 19.12.2001
(73) Proprietor: Miki, Keizaburo, Naka-gun, Kanagawa 255-0004 (JP)
(72) Inventor: MIWA, Johji, Isogo-ku, Yokohama-shi Kanagawa 235-0034 (JP); ISOWA, Nozomu, Shima-cho, Shima-gun, Mie 517-0704 (JP); MIKI, Keizaburo, Kanagawa 255-0004 (JP)
(74) Representative: Walton, Seán Malcolm
(86) International application number: PCT/JP2000/001060
(87) International publication number: WO 2000/049862

(56) References cited:
- WO-A-95/20872
- WO-A-96/15662
- WO-A-96/36716
- WO-A-97/29319
- US-A- 5 969 211
- CADORET J -P ET AL: "Microinjection of bivalve eggs: Application in genetics." MOLECULAR MARINE BIOLOGY AND BIOTECHNOLOGY, vol. 6, no. 1, 1997, pages 72-77, XP001079818 ISSN: 1053-6426
- LEONARDO ELECTRONIC ALMANAC, [Online] vol. 6, no. 11, December 1998 (1998-12), XP002202618 ISSN: 1071-4391 Retrieved from the Internet: <URL:http://www.ekac.org/transgenic.html> [retrieved on 2002-06-06]
- KENDALL J M ET AL: "Aequorea victoria bioluminescence moves into an exciting new era" TRENDS IN BIOTECHNOLOGY, ELSEVIER PUBLICATIONS, CAMBRIDGE, GB, vol. 16, no. 5, 1 May 1998 (1998-05-01), pages 216-224, XP004117786 ISSN: 0167-7799
- SIN FRANK Y T: "Transgenic fish." REVIEWS IN FISH BIOLOGY AND FISHERIES, vol. 7, no. 4, December 1997 (1997-12), pages 417-441, XP001080075 ISSN: 0960-3166
- TSAI H.-J. ET AL. TRANSGENIC RESEARCH vol. 6, 1997, pages 85 - 95, XP002926001
- LU J.-K. ET AL. PROC. NATL. ACAD. SCI. USA vol. 93, 1996, pages 3482 - 3486, XP002926002
- POWERS D.A. ET AL. MOLECULAR MARINE BIOLOGY AND BIOTECHNOLOGY vol. 4, no. 4, 1995, pages 369 - 375, XP002926003
- CADORET J.-P. ET AL. JOURNAL OF BIOTECHNOLOGY vol. 56, 1997, pages 183 - 189, XP002926004
- SATO M. ET AL. ANIMAL BIOTECHNOLOGY vol. 5, no. 1, 1994, pages 19 - 31, XP002926005

## Description

### Technical Field

The present invention relates to a transgenic mollusk and a method for producing the same.

### Background Art

By the conventional pearl culture, only several kinds of naturally-occurring pearls can be produced, and their color tones are modified by staining or the like after production of the pearls. Such modification of color after production of the pearls is likely to fade and it is almost impossible to change the color tone to the desired one because the nacreous layer is hard.

### Disclosure of the Invention

Providing a transgenic pearl shell which has an ability to produce a colored pearl is advantageous in the field of pearl culture. However, to say nothing of a transgenic pearl shell having such a useful property, in the entire phylum Mollusca, a transgenic mollusk in accordance with the present invention as defined in the claims has not been produced.

WO 96/15662 describes methods of enhancing the growth of pearl producing mollusks through the exposure of the mollusk to a growth enhancing agent. Transgenic mollusks capable of expression of growth enhancing agents are also described. Modification of color is not disclosed.

Cadoret et al., Molecular Marine Biology and Biotechnology, Vol. 6, No: 1, 1997, describes expression of a β-galactosidase reporter gene in mollusks. Modification of pearl color is not disclosed.

WO 96/36716 describes transgenic plants exhibiting altered flower color. Sin, Reviews in Fish Biology and Fisheries, Vol. 7, 1997, describes transgenic fish expressing green fluorescent protein.

WO 95/20872; Lu et al., Proceedings of the National Academy of Sciences USA, Vol. 93, 1996; Powers et al., Molecular Marine Biology and Biotechnology, Vol. 4, No. 4, 1995; and Cadoret et al., Journal of Biotechnology, Vol. 56, 1997, all describe expression of reporter genes relating to coloring in mollusks. Modification of pearl color is not disclosed.

Accordingly, an object of the present invention is to provide a transgenic mollusk which can express a desired foreign gene and a method for producing the same.

The present inventors intensively studied to succeed in producing a transgenic mollusk as defined in the claims by microinjecting into gonad of male and female mollusks a recombinant vector into which a fusion gene to be introduced or a nucleic acid containing the fusion gene is inserted, and crossing the male and female; or by introducing into fertilized eggs or embryos of a mollusk to be transformed a recombinant vector into which a nucleic acid including a promoter having a promoter activity in said mollusk and said fusion gene located at a downstream region of said promoter is inserted, thereby completing the present invention.

That is, the present invention provides a transgenic mollusk as defined in the claims. The present invention also provides a method for producing the above-mentioned transgenic mollusk according the present invention, comprising microinjecting into gonad of male and/or female of mollusk a recombinant vector into which a fusion gene to be introduced or a nucleic acid containing the fusion gene is inserted; crossing the male and female to produce individuals of first generation; and selecting therefrom (an) individual(s) which express(es) the fusion gene. The present invention further provides a method for producing the above-mentioned transgenic mollusk according to the present invention, comprising introducing into unfertilized eggs, fertilized eggs or embryos of a mollusk to be transformed a recombinant vector into which a nucleic acid including a promoter having a promoter activity in the mollusk and the fusion gene located at a downstream region of the promoter and functionally linked to the promoter is inserted; developing the unfertilized eggs, fertilized eggs or embryos to individuals; and selecting therefrom (an) individual(s) which express(es) the desired gene.

The present invention also provides a method of producing a pearl of modified color from the transgenic pearl shell mollusk into which a foreign gene relating to coloring has been introduced, which foreign gene is expressed in a pearl shell mollusk, wherein the color of the mantle of said transgenic mollusk is modified by said forein gene relating to coloring; and collecting the pearl of modified colour.

By the present invention, a transgenic mollusk which can express a fusion gene was first provided. The present invention made it possible to produce various mollusks, such as a pearl shell yielding a colored pearl, which are industrially useful.

### Best Mode for Carrying out the Invention

The transgenic mollusk according to the present invention is a pearl shell mollusk, such as *Pinctada fucata Martensii, Pinctada maxima* and *Pinctada margaritifera.*

The fusion gene to be introduced into the mollusk comprises a foreign gene relating to coloring (excluding a gene giving resistance to a virus), such as green fluorescence protein (GFP) gene, anthocyanin gene, fluorescent luciferase gene, β-galactosidase gene and phosphatase gene. The term "foreign gene relating to coloring" includes, as apparent from the above-mentioned examples, not only genes encoding pigments (including fluorescent pigments), but also genes encoding substances which participate in pigment-formation reactions in the body, such as genes encoding enzymes which catalyze reactions forming pigments in the body, providing the foreign gene can be expressed in a pearl shell mollusk.

The transgenic mollusk according to the present invention may be produced as follows: In the first method, a recombinant vector into which a fusion gene to be introduced or a nucleic acid containing the foreign gene is inserted is microinjected into gonad of male and/or female of mollusk; the male and female are crossed to produce individuals of first generation; and (an) individual(s) which express(es) the fusion gene is(are) selected therefrom.

What inserted into the recombinant vector to be microinjected may be the fusion gene alone or may be a nucleic acid containing the fusion gene. Examples of such a nucleic acid include those containing a promoter which shows promoter activity in the mollusk cells, which promoter is located at an upstream region of the fusion. The foreign gene relating to coloring is fused with at least one of: a prison protein gene, a mantle protein gene, a nacreous layer protein gene, a prism layer skeleton protein gene and calcium carbonate-crystallizing enzyme gene. Examples of the vector include vectors for animal cells such as adenovirus vectors, retrovirus vectors and the like. These vectors are commercially available, and commercially available vectors may be employed.

The method for microinjecting the above-mentioned recombinant vector to the gonad of a mollusk will now be described. Basically, the microinjection may be carried out by directly injecting a solution containing the recombinant vector into the gonad through an injection needle. The medium of the solution for microinjection may be a buffer such as TE buffer. The concentration of the recombinant vector in the solution may preferably be about 2 to 200 µg/ml, more preferably about 5 to 10 µg/ml. The amount of the solution to be injected may preferably be about 10 to 50 µl per site, and it is preferred to inject the solution into 2 to 4 sites in a gonad in case of either ovary or testis.

After the microinjection, the mollusk is left to stand at 10 to 25°C, preferably at 15 to 20°C for 24 to 72 hours, preferably 24 to 48 hours, and then the male and the female which received the microinjected solution are crossed. Although the crossing may be carried out by natural crossing, to securely attaining the crossing with high reproducibility, it is preferred to carry out the crossing by artificial fertilization. Artificial fertilization may be carried out basically by adding the sperms from the testis which underwent the microinjection to mature eggs in the ovary of the female that underwent the microinjection. Although it is preferred to conduct the microinjection on the gonads of both of the male and female to be crossed, a transgenic mollusk may be produced even if the gonad of only one of the male and female is subjected to the microinjection. The artificial fertilization *per se* of the mollusk may be carried out by the method described in Dev Biol 1994, 163(1): 162-174 (this paper is herein incorporated by reference) or the like.

Growing individuals from the fertilized eggs may easily be carried out by incubating the fertilized eggs in sea water or artificial sea water at a temperature within the growing temperature range of the mollusk.

Transgenic individuals are then selected from the obtained individuals. This may be carried out by checking whether the desired foreign gene introduced exists in the mollusk cells by Southern blotting, and further checking whether the foreign gene is expressed in the mollusk cells by Northern blotting. Southern blotting and Northern blotting *per se,* as well as methods for preparing the samples therefor are well-known in the art, and are described in, for example, Nakayama and Nishigata, "Bio Experiments Illustrated - ② Base of Gene Analysis", Shujunsha (1995).

To establish a transgenic line, it is preferred to cross the male and female of the individuals of the first generation, which were confirmed to be transgenic, thereby obtaining the individuals of the second generation, and to select the transgenic individuals therefrom in the same manner as mentioned above. Further, by producing the third or more generation, the transgenic line may be established more securely.

In the second method for producing the transgenic pearl shell mollusk, a recombinant vector into which a nucleic acid including a promoter having a promoter activity in the mollusk and the fusion gene located at a downstream region of the promoter and functionally linked to the promoter is inserted is introduced into unfertilized eggs, fertilized eggs or embryos of a mollusk to be transformed; the unfertilized eggs, fertilized eggs or embryos are developed to individuals; and (an) individual(s) which express(es) the desired gene is (are) selected therefrom.

The promoter showing promoter activity in the pearl shell mollusk to be transformed may be any promoter which shows promoter activity in the mollusk to be transformed. Examples of the promoter include actin gene promoter and heat shock protein gene promoter, but the promoter is not restricted thereto. The term "functionally linked" in the phrase "the fusion gene located at a downstream region of the promoter and functionally linked to the promoter" means that the fusion gene is linked to the promoter such that the reading frame thereof is so adjusted as to be controlled by the promoter. In this case, it is possible to express the fusion gene as a fused protein by ligating the foreign gene relating to coloring to the downstream end of a structural gene selected from: a prism protein gene, a mantle protein gene, a nacreous layer protein gene, a prism layer skeleton protein gene and a calcium carbonate-crystallising gene such that the reading frame of the foreign gene is coincide with that of the structural gene which structural genre is functionally linked to the promoter. The method for functionally link a structural gene to a promoter at a site downstream of the promoter is well-known in the art. The recombinant vector may be prepared in the same manner as in the first method.

Then the prepared recombinant vector is introduced into unfertilized eggs, fertilized eggs or embryos, preferably unfertilized eggs of the mollusk. This may be carried out, for example, as follows: A vector solution having a concentration of about 100 to 200 mg/ml is placed in a petri dish or well and the unfertilized eggs, fertilized eggs or embryos are immersed therein. The eggs or embryos thus immersed are then keenly injured only in a moment with a micropipet for microinjection (not restricted thereto) so as to inject the vector solution into the eggs or the embryos. At this time, it is important to form a hole by injuring without bursting the cells or lethally injuring the cells. Individuals may be obtained from the eggs or embryos into which the recombinant vector was introduced by the same method as in the first method, and a transgenic line may be established by selecting the transgenic individuals as in the first method.

Individuals may be obtained from unfertilized eggs by carrying out artificial fertilization immediately after or simultaneously with the above-mentioned operation, and then developing the individuals therefrom as in the first method.

### Examples

The present invention will now be described by way of examples. It should be noted that the present invention are not restricted to the examples below.

### Reference Example 1 Preparation of Transgenic Pinctada fucata Martensii into which Human Interferon α Gene is Introduced

Human or mouse interferon α gene (commercially available from BBL and RDS, respectively) was inserted into an adenovirus vector (commercially available from TAKARA SHUZO, Takara Adenovirus Expression Vector Kit) to obtain recombinant vectors. This operation was carried out concretely as follows: Each of the above-mentioned commercially available interferon α genes was inserted into the *Swa* I site of a cosmid vector (pAxcwt (44,741 bp), Niwa, M. et al., (1991) Gene 108, 193, this cosmid vector is included in the above-mentioned commercially available Adenovirus Expression Vector Kit). The cosmid vector having the inserted gene and the above-mentioned commercially available adenovirus-derived DNA-TPC (Miyake, S. et al., (1996), Proc. Natl. Acad. Sci. USA 93 1320) digested with the above-mentioned restriction enzyme were co-transfected into 293 cells (human fetal kidney cell, commercially available from DAINIPPON PHARMACEUTICAL CO., LTD). The 293 cells were cultured in 10% FCS-containing DMEM medium under 5% CO₂ at 37°C until 100% confluency is achieved, and 10 µg of the above-mentioned cosmid vector DNA and 5 µg of the restriction enzyme-treated DNA-TPC were mixed on a petri dish with a diameter of 6 cm. The transfection was carried out by the calcium phosphate method. The cells after the co-transfection were cultured at 37°C under 5% CO₂ for 24 hours, and the fragment of grown recombinant adenovirus was recovered. The collected fragment was injected into ovaries of *Pinctada fucata Martensii* in an amount of 100 to 200 mg DNA/ovary. Sperms (twice amount of eggs) were mixed with the eggs in a test tube to carry out fertilization. The resulting eggs were cultured in sea water at 25°C for 24 days to obtain young shells. In 31 young shells among 200 young shells, fluorescence (FITC) of a DNA probe for detecting interferon gene was observed. The DNAs of these young shells were purified and existence of the sequence was confirmed with the same DNA probe. These shells were continued to be cultivated.

It has been reported that adductor muscle is colored in red by virus infection. In 18 adult shells among 20 shells which were confirmed to have the interferon gene, this change of color to red was not observed.

On the other hand, each of the recombinant adenovirus vectors obtained as described above was transfected into Hela cells and grown therein. The transfection into Hela cells and proliferation of the virus were carried out by the method described in Nature 1995, 374(6523):660-662. The recombinant vector DNA was recovered from the Hela cells by a conventional method and the obtained DNA was dissolved in 10 mM Tris-HCl (pH7.5), 1 mM EDTA or 20 mM potassium phosphate, 3 mM potassium citrate and 2% PEG-6000 (pH7.5) to a concentration of 50 to 100 µg DNA/ml to obtain a solution for microinjection. This solution was microinjected into ovaries of female *Pinctada fucata Martensii* and testes of male *Pinctada fucata Martensii.* The amount of the injected solution was 100 µg in terms of DNA per site, and the solution was injected from three sites per ovary or testis. Twenty four to forty eight hours later, artificial fertilization was performed using sperms from the testes and mature eggs from the ovaries. The artificial fertilization was carried out concretely as follows: Testis was dissected from each male *Pinctada fucata Martensii* and ovary was dissected from each female *Pinctada fucata Martensii,* and sperms and eggs were taken out therefrom into a test tube at a ratio of 1:2, followed by mixing the sperms and the eggs. The obtained fertilized eggs were incubated in sea water for 2 to 3 weeks at 25°C to obtain individuals of *Pinctada fucata Martensii* of the first generation from the fertilized eggs. Total DNAs were collected from the gonad of each of the obtained *Pinctada fucata Martensii,* and Southern blotting was carried out by a conventional method (Bio Experiments Illustrated, *supra*) using human interferon α gene as a probe. Further, total mRNAs were collected from block of visceral organs and from adductor muscle cells, and Northern blotting was carried out by a conventional method (Bio Experiments Illustrated, *supra*) using human interferon α gene as a probe.

Using mature eggs and sperms collected from male and female individuals which were Southern blot positive and Northern blot positive, artificial fertilization was carried out in the same manner as described above, and individuals of the second generation were obtained as described above. By carrying out Southern blot and Northern blot as described above, 3 lines of transgenic *Pinctada fucata Martensii* into which human interferon α gene was introduced were selected.

The thus prepared transgenic *Pinctada fucata Martensii* and the conventional *Pinctada fucata Martensii* were cultivated for 180 days in a sea region which was thought to be contaminated with viruses and in a sea region which was thought to be not contaminated with viruses judging from the state of cultivated *Pinctada fucata Martensii,* and the lethalities of the groups were compared. The results are shown in Table 1.

**Table 1**

| Effect of Transfection of Human Interferon α Gene (cultivated for 360 days) | | |
|---|---|---|
| *Pinctada fucata Martensii* | Lethality (%) | |
| | Non-contaminated Sea Water | Contaminated Sea Water |
| Conventional Species | 10 | 95 |
| Line 1 | 6 | 30 |
| Line 2 | 1 | 35 |
| Line 3 | 7 | 32 |

As apparent from Table 1, while the lethalities of the transgenic *Pinctada fucata Martensii* according to the present invention (Lines 1-3) in the non-contaminated sea region were similar to that of the conventional species, the lethalities of the former in the contaminated sea region were much lower than that of the conventional species. These results clearly indicate the lethality-decreasing effect by transfection of human interferon α gene.

### Reference Example 2 Preparation of Transgenic Pinctada fucata Martensii into which Human Interferon β Gene is Introduced

The operations as in Reference Example 1 were repeated except that human interferon β gene (commercially available from BBL, HIGASHI, Y. et al. (1983) J. Biol. Chem. 258:92) was used in place of human interferon α gene, and that a 5'-end fragment of the human interferon β gene, which fragment has a size of 40 to 50 bp and labeled with fluorescent FITC was used as the probe in the Southern blot and Northern blot, to prepare transgenic *Pinctada fucata Martensii* (second generation) into which human interferon β gene was introduced.

The effectiveness of the transfection of the human interferon β gene was examined in the same as in Reference Example 1. The results are shown in Table 2.

**Table 2**

| Effect of Transfection of Human Interferon β Gene (cultivated for 180 days) | | |
|---|---|---|
| *Pinctada fucata Martensii* | Lethality (%) | |
| | Non-contaminated Sea Water | Contaminated Sea Water |
| Conventional Species | 11 | 94 |
| Line 1 | 10 | 28 |
| Line 2 | 14 | 22 |
| Line 3 | 12 | 15 |

As in Reference Example 1, the effect of the transfection of human interferon β gene was clearly observed.

### Example 1 Preparation of Pinctada fucata Martensii Having Green Fluorescent Protein (GFP) Gene (1)

Full length GFP gene (Science 1994, 263:802-805; GenBank No. U53602, commercially available from WAKO PURE CHEMICAL INDUSTRIES, LTD.) was inserted into the adenovirus vector in the same manner as in Reference Example 1 (the cells used for the growth of the virus were 293 cells). The obtained GFP gene-containing recombinant vector was dissolved in TE buffer to a concentration of 100 mg/ml, and the solution was microinjected into the ovaries of *Pinctada fucata Martensii* as in Reference Example 1. Thereafter, the same operations as in Reference Example 1 were repeated (except that the probe used in the Southern blot and Northern blot was GFP gene) to prepare transgenic *Pinctada fucata Martensii* (second generation).

*Various tissues of the obtained transgenic Pinctada fucata Martensii were* examined for fluorescence with a fluorescence microscope. The results are shown in Table 3 below. In Table 3, "+" means that fluorescence was observed, and the more the number of "+", the stronger the emitted fluorescence.

**Table 3 Tissues in Which Emitted Fluorescence Was Observed**

| Transgenic *Pinctada fucata Martensii* | Block of Bisceral Organs | Atrium | Adductor Muscle | Mantle | Leg |
|---|---|---|---|---|---|
| Line 1 | +++ | ++ | ++ | +++ | ++ |
| Line 2 | + | + | + | + | +++ |
| Line 3 | +++ | +++ | ++ | +++ | ++ |

### Example 2 Preparation of Pinctada fucata Martensii Having Green Fluorescent Protein (GFP) Gene (2)

Prism protein of pearl shell is an important protein constituting pearls. In this Example, it was tried to make pearls emit fluorescence by themselves by fusing the prism protein gene with the green fluorescence protein gene.

Prism protein gene of *Pinctada fucata Martensii* (Nature 1997, 387(6633):563-564, hereby incorporated by reference); GenBank No. D860/3) together with its upstream region from its initiation codon to an upstream site thereof by 5 kb was cloned, and GFP gene was fused with the promoter, followed by inserting the resultant to an adenovirus vector. The fusion gene comprising the prism protein gene (containing the promoter) and the GFP gene was prepared by the method described by M. Chalfie et al., Science 1994, 263:802-805 hereby incorporated by reference). That is, at 10nt from the initiation codon of the above-mentioned prism protein gene including the upstream region from the initiation codon by 5 kb, a linker (polyT) with a size of 9nt, 10nt or 11nt was ligated. On the other hand, to the 5'-end of a commercially available GFP gene, polyA linker with a size of 9nt, 10nt or 11 nt was ligated. These DNA fragments were hybridized to obtain a fusion gene. Using restriction enzymes NHe I and Eco RI, the obtained fusion gene was inserted into the same adenovirus vector as used in Reference Example 1. Thereafter, in the same manner as in Reference Example 1, transgenic *Pinctada fucata Martensii* were obtained into which the fusion gene comprising the prism protein gene and the GFP gene was introduced. The mantle tissues of the obtained transgenic *Pinctada fucata Martensii* were observed with a fluorescence microscope to confirm emission of fluorescence.

The transgenic *Pinctada fucata Martensii* obtained in this Example contained the GFP gene at a site downstream of the promoter and the structural gene of the prism protein gene, and emission of fluorescence by expression of the fusion gene was observed. Therefore, if pearls are produced by these transgenic *Pinctada fucata Martensii,* it is thought that pearls which emit fluorescence are formed because GFP is fused with the prism protein constituting the pearls.

### Example 3 Preparation of Pinetada fucata Martensii Having Green Fluorescent Protein (GFP) Gene (3)

Mantle protein is an important protein constituting pearls like prism protein. The same operations as in Example 2 were repeated except that the mantle protein gene (Nature 1997, 387(6633):563-564 (hereby incorporated by reference), GenBank No. 86074) to prepare transgenic *Pinctada fucata Martensii* into which a fusion gene comprising mantle protein gene and the GFP gene was introduced. The fusion of the *Pinctada fucata Martensii* mantle protein gene and the GFP gene, and the insertion of the fusion gene into the adenovirus vector were carried out concretely as follows: As in Example 2, polyT with a size of 9nt, 10nt or 11 nt was ligated to the 15nt from the initiation codon of a DNA fragment including the upstream region of the initiation codon of the mantle protein to an upstream site thereof by about 5 kb. On the other hand, polyA with a size of 9nt, 1 0nt or 11 nt was ligated to the GFP gene as used in Example 2. These DNA fragments were hybridized to prepare a fusion gene. Using the thus obtained fusion gene, the same operations as in Example 2 were repeated to obtain transgenic *Pinctada fucata Martensii.*

The mantle tissues of the obtained transgenic *Pinctada fucata Martensii* were observed with a fluorescence microscope to confirm emission of fluorescence.

The transgenic *Pinctada fucata Martensii* obtained in this Example contained the GFP gene at a site downstream of the promoter and the structural gene of the mantle protein gene, and emission of fluorescence by expression of the fusion gene was observed. Therefore, if pearls are produced by these transgenic *Pinctada fucata Martensii,* it is thought that pearls which emit fluorescence are formed because GFP is fused with the mantle protein constituting the pearls.

### Example 4 Introduction of GFP Gene or LacZ Gene by Promoter Trap Method

Basically, the method by I. A. Hope, Development, Vol. 113, 339-408(1991) (hereby incorporated by reference) was followed. Firstly, by the method described in Example 1, GFP gene (10 to 50 mg DNA/gonad) was introduced into testes and ovaries, and then fertilization was carried out in test tubes. The eggs were cultured in sea water at 25°C for 3 weeks to obtain young shells. About 5 to 15% of the young shells showed the coloring characteristic to GFP. These were cultivated in a cultivation bath for 12 months to obtain adult shells. The adult shells were anatomized and the organs which especially well colored were separated, followed by extraction of DNAs from the organs. In the extracted DNAs, the promoter controlling expression of the GFP gene was analyzed. Promoters of protein synthetases and of enzymes in the adductor muscle were trapped.

At a site downstream of the newly isolated sequences containing the promoter region, GFP gene or LacZ gene was inserted, and each of the obtained genes was inserted into the Swa I restriction site (between the cytomegalovirus enhancer sequence and rabbit β-globin polyA signal) of an expression vector pAxCAwt (TAKARA Adenovirus Expression Vector Kit). A solution of each of the obtained recombinant vectors with a concentration of about 100 to 200 mg/ml was prepared in a petri dish, and non-fertilized eggs were immersed therein. The eggs thus immersed are then keenly injured only in a moment with a micropipet for microinjection so as to inject the vector solution into the eggs. Thereafter, the same operations as in Reference Example 1 were repeated to obtain transgenic *Pinctada fucata Martensii.* The amount of expression of each gene was spectroscopically determined.

The obtained transgenic *Pinctada fucata Martensii* were examined for self emission of fluorescence (in case of introducing GFP gene) or for staining by coloring substrate XG (in case of introducing LacZ gene), and emission of fluorescence or staining was confirmed in various tissues of *Pinctada fucata Martensii.*

### Industrial Availability

By the present invention, transgenic mollusks having the desired properties may be provided. Thus, for example, industrially useful mollusks such as *Pinctada fucata Martensii* which yield colored pearls may be provided.

## Claims

1. A transgenic pearl shell mollusk capable of producing a pearl of modified colour, into which mollusk a fusion gene has been introduced, said fusion gene comprising a foreign gene relating to coloring, excluding a gene giving resistance to a virus, which foreign gene is expressed in a pearl shell mollusk, fused to at least one of: a prism protein gene, a mantle protein gene, a nacreous layer protein gene, prism layer skeleton protein gene and calcium carbonate-crystallising enzyme gene, wherein the color of the mantle of said transgenic mollusk is modified by said gene foreign gene relating to coloring.

2. The transgenic pearl shell mollusk according to claim 1, wherein the said foreign gene relating to coloring is fused to either a prism protein gene or a mantle protein gene.

3. The transgenic pearl shell mollusk according to claim 1, wherein said foreign gene relating to coloring is green fluorescence protein gene, anthocyanin gene, fluorescent luciferase gene, β-galactosidase gene or phosphatase gene.

4. The transgenic pearl shell mollusk according to claim 1, wherein said foreign gene relating to coloring is a gene encoding green fluorescence protein, and at least one of the tissues of said transgenic mollusk emits fluorescence.

5. A method for producing the transgenic pearl shell mollusk according to any one of claims 1 to 4, comprising microinjecting into gonad of a male and/or a female pearl shell mollusk a recombinant vector into which said fusion gene to be introduced is inserted, said fusion gene comprising a foreign gene relating to coloring, excluding a gene giving resistance to a virus, which foreign gene is expressed in a pearl shell mollusk, fused to at least one of a prism protein gene, a mantle protein gene, a nacreous layer protein gene, prism layer skeleton protein gene and calcium carbonate-crystallising enzyme gene; crossing said male and female to produce individuals of first generation; and selecting therefrom (an) individual(s) which express(es) said desired gene.

6. The method according to claim 5, further comprising crossing male and female of said selected individuals of first generation, which express said fusion gene to produce individuals of second generation, and selecting therefrom (an) individual(s) which express(es) said desired gene.

7. A method for producing the transgenic pearl shell mollusk according to any one of claims 1 to 4, comprising introducing into unfertilized eggs, fertilized eggs or embryos of a mollusk to be transformed a recombinant vector into which a nucleic acid including a promoter having a promoter activity in said mollusk and said fusion gene located at a downstream region of said promoter and functionally linked to said promoter is inserted, said fusion gene comprising a foreign gene relating to coloring, excluding a gene giving resistance to a virus, which foreign gene is expressed in a pearl shell mollusk, fused to at least one of a prism protein gene, a mantle protein gene, a nacreous layer protein gene, prism layer skeleton protein gene and calcium carbonate-crystallising enzyme gene; developing said unfertilized eggs, fertilized eggs or embryos to individuals; and selecting therefrom (an) individual(s) which express(es) said desired gene.

8. The method according to any one of claims 5 to 7, wherein said foreign gene relating to coloring is fused to either a prism protein gene or a mantle protein gene.

9. The method according to any one of claims 5 to 7, wherein said foreign gene relating to coloring is green fluorescence protein gene, anthocyanin gene, fluorescent luciferase gene, galactosidase gene or phosphatase gene.

10. A method of producing a pearl of modified color from the transgenic pearl shell mollusk into which a foreign gene relating to coloring has been introduced, which foreign gene is expressed in a pearl shell mollusk, wherein the color of the mantle of said transgenic mollusk is modified by said forein gene relating to coloring; and collecting the pearl of modified colour.

11. Use of a transgenic pearl shell mollusk according to any one of claims 1 to 4 in the production of a pearl of modified colour.

## Patentansprüche

1. Transgene Perlenmuschel-Molluske, die zur Bildung einer Perle mit modifizierter Farbe in der Lage ist, wobei in die Molluske ein Fusionsgen eingeführt wurde, worin das Fusionsgen ein mit der Farbgebung zusammenhängendes fremdes Gen, ausgenommen ein Resistenz gegen ein Virus verleihendes Gen, umfasst, wobei das fremde Gen in einer Perlenmuschel-Molluske exprimiert wird, und zwar an zumindest eines der Folgenden fusioniert: ein Prismenproteingen, ein Mantelproteingen, ein Perlmuttschichtproteingen, ein Prismenschichtskelettproteingen und ein Calciumcarbonat-Kristallisierungsenzymgen, worin die Farbe des Mantels der transgenen Molluske durch das mit der Farbgebung zusammenhängende fremde Gen modifiziert ist.

2. Transgene Perlenmuschel-Molluske nach Anspruch 1, worin das mit der Farbgebung zusammenhängende fremde Gen entweder an ein Prismenproteingen oder ein Mantelproteingen fusioniert ist.

3. Transgene Perlenmuschel-Molluske nach Anspruch 1, worin das mit der Farbgebung zusammenhängende fremde Gen ein Gen eines grün fluoreszierenden Proteins, ein Anthocyaningen, ein Gen einer fluoreszierenden Luciferase, ein β-Galactosidasegen oder ein Phosphatasegen ist.

4. Transgene Perlenmuschel-Molluske nach Anspruch 1, worin das mit der Farbgebung zusammenhängende fremde Gen ein Gen ist, das für ein grün fluoreszierendes Protein kodiert, und zumindest eines der Gewebe der transgenen Molluske Fluoreszenz emittiert.

5. Verfahren zur Herstellung einer transgenen Perlenmuschel-Molluske nach einem der Ansprüche 1 bis 4, umfassend das Mikroinjizieren eines rekombinanten Vektors, in den das einzuführende Fusionsgen insertiert ist, in die Gonade einer männlichen und/oder weiblichen Perlenmuschel-Molluske, wobei das Fusionsgen ein mit der Farbgebung zusammenhängendes fremdes Gen, ausgenommen ein Resistenz gegen ein Virus verleihendes Gen, umfasst, wobei das fremde Gen in einer Perlenmuschel-Molluske exprimiert wird, und zwar an zumindest eines aus einem Prismenproteingen, einem Mantelproteingen, einem Perlmuttschichtproteingen, einem Prismenschichtskelettproteingen und einem Calciumcarbonat-Kristallisierungsenzymgen fusioniert; das Kreuzen des männlichen und weiblichen Tiers, um Individuen einer ersten Generation herzustellen; und das Auswählen eines oder mehrerer Individuen daraus, die das gewünschte Gen exprimieren.

6. Verfahren nach Anspruch 5, umfassend weiters das Kreuzen eines männlichen und weiblichen Tiers aus den ausgewählten Individuen der ersten Generation, welche das Fusionsgen exprimieren, um Individuen einer zweiten Generation herzustellen, und das Auswählen eines oder mehrerer Individuen daraus, die das gewünschte Gen exprimieren.

7. Verfahren zur Herstellung einer transgenen Perlenmuschel-Molluske nach einem der Ansprüche 1 bis 4, umfassend das Einführen eines rekombinanten Vektors, in den eine Nucleinsäure, die einen Promotor mit Promotoraktivität in dieser Molluske und das Fusionsgen in einer stromab gelegenen Region des Promotors und funktionell an diesen Promotor gebunden umfasst, insertiert ist, in unbefruchtete Eier, befruchtete Eier oder Embryos einer zu transformierenden Molluske, wobei das Fusionsgen ein mit der Farbgebung zusammenhängendes fremdes Gen, ausgenommen ein Resistenz gegen ein Virus verleihendes Gen, umfasst, wobei das fremde Gen in einer Perlenmuschel-Molluske exprimiert wird, und zwar an zumindest eines aus einem Prismenproteingen, einem Mantelproteingen, einem Perlmuttschichtproteingen, einem Prismenschichtskelettproteingen und einem Calciumcarbonat-Kristallisierungsenzymgen fusioniert; das Entwickeln der unbefruchteten Eier, befruchteten Eier oder Embryos zu Individuen; und das Auswählen eines oder mehrerer Individuen daraus, die das gewünschte Gen exprimieren.

8. Verfahren nach einem der Ansprüche 5 bis 7, worin das mit der Farbgebung zusammenhängende fremde Gen entweder an ein Prismenproteingen oder ein Mantelproteingen fusioniert ist.

9. Verfahren nach einem der Ansprüche 5 bis 7, worin das mit der Farbgebung zusammenhängende fremde Gen ein Gen eines grün fluoreszierenden Proteins, ein Anthocyaningen, ein Gen einer fluoreszierenden Luciferase, ein β-Galactosidasegen oder ein Phosphatasegen ist.

10. Verfahren zur Herstellung einer Perle mit modifizierter Farbe aus der transgenen Perlenmuschel-Molluske, in die ein mit der Farbgebung zusammenhängendes fremdes Gen eingeführt wurde, wobei das fremde Gen in einer Perlenmuschel-Molluske exprimiert wird, worin die Farbe des Mantels der transgenen Molluske durch das mit der Farbgebung zusammenhängende fremde Gen modifiziert ist; und das Entnehmen der Perle mit modifizierter Farbe.

11. Verwendung einer transgenen Perlenmuschel-Molluske nach einem der Ansprüche 1 bis 4 zur Herstellung einer Perle mit modifizierter Farbe.

## Revendications

1. Mollusque à coquille nacrée transgénique capable de produire une perle de couleur modifiée, dans lequel un gène de fusion a été introduit, ledit gène de fusion comprenant un gène étranger lié à la coloration, à l'exclusion d'un gène conférant une résistance à un virus, lequel gène étranger est exprimé dans un mollusque à coquille nacrée, fusionné à au moins un parmi: un gène de protéine de prisme, un gène de protéine de manteau, un gène de protéine de couche nacrée, un gène de protéine de squelette de couche prismatique et un gène d'enzyme de cristallisation du carbonate de calcium, la couleur du manteau dudit mollusque transgénique étant modifiée par ledit gène étranger lié à la coloration.

2. Mollusque à coquille nacrée transgénique selon la revendication 1, dans lequel ledit gène étranger lié à la coloration est fusionné à un gène de protéine de prisme ou à un gène de protéine de manteau.

3. Mollusque à coquille nacrée transgénique selon la revendication 1, dans lequel ledit gène étranger lié à la coloration est le gène de la protéine verte fluorescente, le gène de l'anthocyanine, le gène de la luciférase fluorescente, le gène de la β-galactosidase ou le gène de la phosphatase.

4. Mollusque à coquille nacrée transgénique selon la revendication 1, dans lequel ledit gène étranger lié à la coloration est un gène codant la protéine verte fluorescente et au moins un des tissus dudit mollusque transgénique émet une fluorescence.

5. Procédé pour produire le mollusque à coquille nacrée transgénique selon l'une quelconque des revendications 1 à 4, comprenant la micro-injection dans la gonade d'un coquillage à coquille nacrée mâle et/ou femelle d'un vecteur recombinant dans lequel ledit gène de fusion à introduire est inséré, ledit gène de fusion comprenant un gène étranger lié à la coloration, à l'exclusion d'un gène conférant une résistance à un virus, lequel gène étranger est exprimé dans un mollusque à coquille nacrée, fusionné à au moins un gène de protéine de prisme, un gène de protéine de manteau, un gène de protéine de couche nacrée, un gène de protéine de squelette de couche prismatique et un gène d'enzyme de cristallisation du carbonate de calcium; le croisement dudit mâle et de ladite femelle pour produire des individus de première génération; et la sélection parmi ceux-ci d'un ou plusieurs individus qui expriment ledit gène souhaité.

6. Procédé selon la revendication 5, comprenant en outre le croisement d'un mâle et d'une femelle desdits individus sélectionnés de première génération, qui expriment ledit gène de fusion, pour produire des individus de deuxième génération, et la sélection parmi ceux-ci d'un ou plusieurs individus qui expriment ledit gène désiré.

7. Procédé pour produire le mollusque à coquille nacrée transgénique selon l'une quelconque des revendications 1 à 4, comprenant l'introduction, dans des oeufs non fertilisés, des oeufs fertilisés ou des embryons d'un mollusque à transformer, d'un vecteur recombinant dans lequel un acide nucléique incluant un promoteur ayant une activité promotrice dans ledit mollusque et ledit gène de fusion situé au niveau d'une région en aval dudit promoteur et lié de manière fonctionnelle audit promoteur est inséré, ledit gène de fusion comprenant un gène étranger lié à la coloration, à l'exclusion d'un gène conférant une résistance à un virus, lequel gène étranger est exprimé dans un mollusque à coquille nacrée, fusionné à au moins un parmi un gène de protéine de prisme, un gène de protéine de manteau, un gène de protéine de couche nacrée, un gène de protéine de squelette de couche prismatique et un gène d'enzyme de cristallisation du carbonate de calcium; le développement desdits oeufs non fertilisés, oeufs fertilisés ou embryons en individus; et la sélection parmi ceux-ci d'un ou plusieurs individus qui expriment lesdits gènes souhaités.

8. Procédé selon l'une quelconque des revendications 5 à 7, dans lequel ledit gène étranger lié à la coloration est fusionné à un gène de protéine de prisme ou à un gène de protéine de manteau.

9. Procédé selon l'une quelconque des revendications 5 à 7, dans lequel ledit gène étranger lié à la coloration est le gène de la protéine verte fluorescente, le gène de l'anthocyanine, le gène de la luciférase fluorescente, le gène de la β-galactosidase ou le gène de la phosphatase.

10. Procédé pour produire une perle de couleur modifiée à partir du mollusque à coquille nacrée transgénique dans lequel un gène étranger lié à la coloration a été introduit, lequel gène étranger est exprimé dans un mollusque à coquille nacrée, dans lequel la couleur du manteau dudit mollusque à coquille nacrée est modifiée par ledit gène étranger lié à la coloration; et la collecte de la perle de couleur modifiée.

11. Utilisation d'un mollusque à coquille nacrée transgénique selon l'une quelconque des revendications 1 à 4 dans la production d'une perle de couleur modifiée.
